# EUROPÄISCHE PATENTANMELDUNG

(11) **EP 4 483 826 A1**
(43) Veröffentlichungstag der Anmeldung: **01.01.2025**
(21) Anmeldenummer: 24185503.0
(22) Anmeldetag: 28.06.2024
(51) Int. Cl.: A61B 18/12, H02M 5/00

(54) **HOCHFREQUENZGENERATOR, SYSTEM, VERFAHREN ZUR ERZEUGUNG EINER HOCHFREQUENTEN RECHTECKSPANNUNG UND VERWENDUNG EINES HOCHFREQUENZGENERATORS**

(30) Priorität: 30.06.2023 DE 102023117342
(71) Anmelder: Karl Storz SE & Co. KG, 78532 Tuttlingen (DE)
(72) Erfinder: HINDING, Thomas, 78532 Tuttlingen (DE)

(57) **Zusammenfassung**

Die Erfindung betrifft einen Hochfrequenzgenerator (10), umfassend einen Primärstromkreis (11), an welchem eine Wechselspannung anlegbar ist, einen Sekundärstromkreis (12), einen Übertrager (13) zur von dem Sekundärstromkreis galvanisch getrennten Kopplung des Primärstromkreises an den Sekundärstromkreis und eine Spannungswandlungseinrichtung (14), welche dazu eingerichtet ist, aus der mittels des Übertragers von dem Primärstromkreis auf den Sekundärstromkreis übertragenen Wechselspannung eine hochfrequente Rechteckspannung zu erzeugen.

## Beschreibung

Die Erfindung betrifft einen Hochfrequenzgenerator, umfassend einen Primärstromkreis, an welchem eine Wechselspannung anlegbar ist, einen Sekundärstromkreis und einen Übertrager zur von dem Sekundärstromkreis galvanisch getrennten Kopplung des Primärstromkreises an den Sekundärstromkreis. Weiter betrifft die Erfindung ein System, umfassend einen Hochfrequenzgenerator und ein Hochfrequenz-Gerät, insbesondere ein elektrochirurgisches Instrument zum Koagulieren und/oder Schneiden von einem biologischen Gewebe. Ferner betrifft die Erfindung ein Verfahren zur Erzeugung einer hochfrequenten Spannung mittels eines Hochfrequenzgenerators. Letztlich betrifft die Erfindung eine Verwendung eines Hochfrequenzgenerators für ein elektrochirurgisches Instrument zum Koagulieren und/oder Schneiden von einem biologischen Gewebe.

Bei der Hochfrequenz-Chirurgie (HF-Chirurgie) oder Elektrochirurgie wird ein hochfrequenter Wechselstrom durch einen menschlichen Körper eines Patienten geleitet, um ein biologisches Gewebe des Körpers durch eine dadurch verursachte Erwärmung gezielt zu schädigen bzw. zu schneiden.

Bei einer minimalinvasiven Endoskopie werden elektrochirurgische Instrumente zur Koagulation oder zum Schneiden verwendet. Dabei ist eine schnelle und zuverlässige Gewebeversiegelung erforderlich. Bereits eine geringe Menge an Blut ist ausreichend, eine Sicht eines Chirurgen zu behindern und einen Eingriff an einem Patienten zu erschweren oder gar unmöglich zu machen. Grundsätzlich wird bei einer Gewebeversiegelung mittels eines hochfrequenten Stroms ein Gewebe erhitzt. Infolge der Erhitzung wird das Gewebe versiegelt. Hierdurch ist es möglich eventuell auftretende Blutungen des Gewebes unmittelbar zu stoppen.

Ein bei der Erhitzung verwendeter Strom muss eine bestimmte Frequenz aufweisen, um eine Reizung von Nerven zu verhindern. Mit anderen Worten sollen die Nerven nicht stimuliert werden. Typischerweise liegen entsprechende Frequenzen im kHz-Bereich.

Um eine benötigte Spannung zu erzielen und eine aus Sicherheitsgründen nötige galvanische Trennung zu erreichen, kann ein Übertrager eingesetzt werden. Der Übertrager wird typischerweise mittels einer Halb- oder Vollbrücke auf einer Primärseite des Hochfrequenzgenerators mit einer Sinusspannung mit einer Frequenz von regelmäßig mindestens 150 kHz betrieben. Auf einer Sekundärseite des Hochfrequenzgenerators kann dann eine galvanisch von der Primärseite getrennte Spannung anliegen, die beispielsweise zum Koagulieren oder Schneiden von einem Gewebe verwendet werden kann. Dabei darf ein zu behandelndes Gewebe nicht zu stark und zu lange erhitzt werden, da es sonst zu einer Karbonisierung oder zu einer Brandschorfbildung kommen kann, die eine weitere unkontrollierbare Blutung auslösen kann. Es ist daher erforderlich, dass der Hochfrequenzgenerator zu einer Signalerzeugung eine Vielzahl von Regelgrößen, insbesondere eine Spannung oder ein Strom, erfasst. Bei bipolaren Systemen ist es zudem erforderlich, dass zusätzlich noch eine Funkerkennung vorhanden ist. Eine genaue oder zuverlässige Messung bzw. Regelung der Spannung ist aufgrund der im Stand der Technik regelmäßig verwendeten Sinusspannung jedoch schwierig.

Die Frequenz wie oben beschrieben wird mittels einer Halb- oder Vollbrücke erzeugt. Die verwendeten Komponenten können aufgrund von hoher Leistung aber nur digital betrieben werden, so dass nur ein Ein- oder Ausschalten erfolgen kann. Ein Übertrager kann idealerweise mit einer Sinusspannung betrieben werden. Ein "hartes" Durchschalten von verwendeten Transistoren, meistens MOSFETs, kann viele Oberwellen hervorrufen, also Störungen, die einerseits bei einer EMV-Prüfung kritisch werden können und andererseits aber auch exakte Messungen von Strom-Spannungs-Phasenverschiebungen erheblich erschweren und ungenau machen können. Bei geringeren Frequenzen kann ein Deltamodulations-Verfahren zum Einsatz kommen. Dieses scheidet bei höheren Frequenzen aber aus, da die Transistoren nicht schnell genug schalten können.

Beispielsweise geht ein mit einem Sinussignal arbeitender Hochfrequenzgenerator aus der am Prioritätstag der vorliegenden Patentanmeldung noch nicht veröffentlichten deutschen Patentanmeldung des Anmelders mit dem amtlichen Aktenzeichen 10 2022 127 935.9 hervor.

Die Offenlegungsschrift US 2013/0110103 A1 offenbart einen Hochfrequenzgenerator zum Erzeugen von Hochfrequenz-Energie für das Schneiden und/oder Koagulieren von biologischem Gewebe. Um die Störanfälligkeit der Aktivierungsschalter des elektrochirurgischen Instruments zu reduzieren, kommen Optokoppler als Signalübertrager zwischen einem Anwendungsteil und einem Zwischenstromkreis zum Einsatz.

US 2020/0093488 A1 zeigt einen Hochfrequenzgenerator für die Elektrochirurgie mit einer Kurzschlusserkennung.

Der vorliegenden Erfindung liegt daher die Aufgabe zugrunde, einen Hochfrequenzgenerator, ein System, umfassend einen Hochfrequenzgenerator und ein Hochfrequenz-Gerät, insbesondere ein elektrochirurgisches Instrument zum Koagulieren und/oder Schneiden von einem biologischen Gewebe, ein Verfahren zur Erzeugung einer hochfrequenten Spannung mittels eines Hochfrequenzgenerators und eine Verwendung eines Hochfrequenzgenerators für ein elektrochirurgisches Instrument zum Koagulieren und/oder Schneiden von einem biologischen Gewebe vorzuschlagen, welcher bzw. welche bzw. welches die Nachteile im Stand der Technik überwindet und insbesondere eine verbesserte Durchführung einer Koagulation oder eines Gewebeschnittes ermöglicht.

Diese Aufgabe wird durch einen Hochfrequenzgenerator mit den Merkmalen des Anspruchs 1, ein System, umfassend einen Hochfrequenzgenerator und ein Hochfrequenz-Gerät, insbesondere ein elektrochirurgisches Instrument zum Koagulieren und/oder Schneiden von einem biologischen Gewebe und mit den Merkmalen des Anspruchs 15, ein Verfahren zur Erzeugung einer hochfrequenten Spannung mittels eines Hochfrequenzgenerators gelöst.

Der erfindungsgemäße Hochfrequenzgenerator umfasst einen Primärstromkreis, an welchem eine Wechselspannung anlegbar ist, einen Sekundärstromkreis, einen Übertrager zur von dem Sekundärstromkreis galvanisch getrennten Kopplung des Primärstromkreises an den Sekundärstromkreis und eine Spannungswandlungseinrichtung, welche dazu eingerichtet ist, aus der mittels des Übertragers von dem Primärstromkreis auf den Sekundärstromkreis übertragenen Wechselspannung eine hochfrequente Rechteckspannung zu erzeugen.

Vorteilhafterweise kann der Hochfrequenzgenerator im Zusammenhang mit einem Hochfrequenz-Gerät, welches insbesondere ein elektrochirurgisches Instrument zum Koagulieren und/oder Schneiden von einem biologischen Gewebe sein kann, eingesetzt werden. Grundsätzlich kann der Hochfrequenzgenerator jedoch auch im Zusammenhang mit beliebigen Hochfrequenz-Anwendungen, welche insbesondere auch abseits des technischen Gebietes der HF-Chirurgie angesiedelt sein können, eingesetzt werden. Nachfolgend wird der Hochfrequenzgenerator und auch das Verfahren insbesondere im Zusammenhang mit einem Hochfrequenz-Gerät bzw. elektrochirurgischen Instrument zum Koagulieren und/oder Schneiden von einem biologischen Gewebe beschrieben, was indes keineswegs beschränkend wirken soll.

Erfindungsgemäß umfasst der Hochfrequenzgenerator einen Primärstromkreis, an welchem eine Wechselspannung anlegbar ist, einen Sekundärstromkreis, an welchem ein Hochfrequenz-Gerät bzw. ein elektrochirurgisches Instrument zum Koagulieren und/oder Schneiden von einem biologischen Gewebe bzw. zumindest ein Elektrodenpaar eines elektrochirurgischen Instruments zum Koagulieren und/oder Schneiden von einem biologischen Gewebe anschließbar sein kann, und einen Übertrager zur von dem Sekundärstromkreis galvanisch getrennten Kopplung des Primärstromkreises an den Sekundärstromkreis. Unter einer galvanischen Trennung (auch galvanische Entkopplung oder Potentialtrennung genannt) wird ein Vermeiden einer elektrischen Leitung zwischen zwei Stromkreisen, zwischen denen Leistung oder Signale ausgetauscht werden sollen, verstanden. Die elektrische Leitung wird dabei bevorzugt durch elektrisch nicht leitfähige Kopplungsglieder aufgetrennt. Bei einer galvanischen Trennung sind elektrische Potentiale voneinander getrennt und die Stromkreise sind dann untereinander potentialfrei. Diese Trennung darf nicht an einer anderen Stelle, beispielsweise über Erdungen, aufgehoben sein. Der Übertrager kann eine Primärwicklung und eine Sekundärwicklung aufweisen. Wird nun an den Primärstromkreis und damit an die Primärwicklung eine Wechselspannung angelegt, so kann in der Primärwicklung ein Wechselstrom ausgebildet werden, welcher ein magnetisches Wechselfeld erzeugen kann, welches seinerseits in der Sekundärwicklung eine dann an dem Sekundärstromkreis anliegende Wechselspannung induzieren kann. Mit anderen Worten kann die an dem Primärstromkreis anlegbare Wechselspannung mittels des Übertragers von dem Primärstromkreis auf den Sekundärstromkreis übertragen werden. Eine Anzahl von Windungen der Sekundärwicklung kann je nach Bedarf größer sein als eine Anzahl von Windungen der Primärwicklung, so dass der Übertrager die Wechselspannung bei der Übertragung der Wechselspannung von dem Primärstromkreis auf den Sekundärstromkreis hochtransformieren kann, um eine ausreichend hohe an dem Sekundärstromkreis anliegende Spannung für eine jeweilige Hochfrequenz-Anwendung, insbesondere zur Gewebeversiegelung, zu erhalten. Jedoch kann die Anzahl der Windungen der Primärwicklung auch mit der Anzahl der Windungen der Sekundärwicklung im Wesentlichen übereinstimmen. Erfindungsgemäß umfasst der Hochfrequenzgenerator weiter eine Spannungswandlungseinrichtung, welche dazu eingerichtet ist, aus der mittels des Übertragers von dem Primärstromkreis auf den Sekundärstromkreis übertragenen Wechselspannung eine hochfrequente Rechteckspannung zu erzeugen, welche an dem Sekundärstromkreis abgreifbar, insbesondere an dem Hochfrequenz-Gerät bzw. elektrochirurgischen Instrument bzw. zwischen Elektroden des Elektrodenpaars anlegbar, sein kann. Mit anderen Worten handelt es sich bei dem Hochfrequenzgenerator um einen Hochfrequenzrechteckgenerator. Dabei erzeugt die Spannungswandlungseinrichtung, welche ein Umrichter sein kann, aus der Wechselspannung eine hochfrequente Rechteckspannung. Die an dem Primärstromkreis anlegbare Wechselspannung kann dabei sinusförmig oder rechteckförmig sein. Die Wechselspannung kann einen geringeren Effektivwert bzw. Scheitelwert als die Rechteckspannung aufweisen. Auch eine Frequenz der Wechselspannung kann geringer sein als eine Frequenz der Rechteckspannung. Eine Form, Größe und Frequenz der Wechselspannung kann derart gewählt sein, dass möglichst wenige Oberwellen erzeugt werden und insbesondere bei dem Übertrager möglichst geringe Leistungsverluste auftreten. Wesentlich ist, dass die Spannungswandlungseinrichtung die Wechselspannung hernach in eine hochfrequente Rechteckspannung umwandelt, welche sodann an dem Sekundärstromkreis abgegriffen, insbesondere an dem Hochfrequenz-Gerät bzw. elektrochirurgischen Instrument bzw. zwischen den Elektroden angelegt und ggf. zum Koagulieren oder zum Gebeschneiden verwendet, werden kann. Der Vorteil der Verwendung eines Rechtecksignals liegt darin, dass dieses exakter als ein Sinussignal vermessen werden kann, wodurch eine Regelung einer erzeugten bzw. abgegriffenen Leistung des Hochfrequenzgenerators, insbesondere einer in das Gewebe eingebrachten Leistung, verbessert und erleichtert werden kann. Dies ermöglicht insbesondere eine Erzielung einer vergleichsweise höheren Qualität bei einer Gewebeversiegelung. Zudem können insbesondere eine Karbonisierung und/oder eine Brandschorfbildung so vermieden werden. Im Ergebnis überwindet der erfindungsgemäße Hochfrequenzgenerator somit die Nachteile im Stand der Technik. Insbesondere ermöglicht der erfindungsgemäße Hochfrequenzgenerator somit eine verbesserte Durchführung einer Koagulation und/oder eines Gewebeschnittes.

Vorteilhafterweise kann also an dem Sekundärstromkreis ein Hochfrequenz-Gerät, insbesondere ein elektrochirurgisches Instrument zum Koagulieren und/oder Schneiden von einem biologischen Gewebe, anschließbar sein, so dass die hochfrequente Rechteckspannung an dem Hochfrequenz-Gerät anlegbar sein kann.

In einer vorteilhaften Ausführungsform der Erfindung kann die Spannungswandlungseinrichtung einen in den Sekundärstromkreis integrierten Gleichrichter zur Umwandlung der mittels des Übertragers von dem Primärstromkreis auf den Sekundärstromkreis übertragenen Wechselspannung in eine gleichgerichtete Arbeitsspannung und zumindest eine in den Sekundärstromkreis integrierte H-Brückenschaltung, an welcher die Arbeitsspannung anlegbar sein kann, umfassen, wobei die H-Brückenschaltung vier elektronische Schalter und einen Brückenzweig, an welchem das Hochfrequenz-Gerät bzw. elektrochirurgische Instrument bzw. Elektrodenpaar anschließbar sein kann, aufweisen kann, wobei die Spannungswandlungseinrichtung zumindest einen Rechteckgenerator umfassen kann, welcher zur Ansteuerung der Schalter eingerichtet sein kann, derart, dass in dem Brückenzweig bzw. zwischen den Elektroden die hochfrequente Rechteckspannung ausbildbar sein kann. Demnach kann die hochfrequente Rechteckspannung an dem Brückenzweig abgegriffen werden. Die H-Brückenschaltung, welche auch H-Brücke, Vollbrücke oder Vierquadrantensteller genannt wird, erlaubt eine besonders einfache Erzeugung der hochfrequenten Rechteckspannung.

In einer Ausführungsform der Erfindung kann die Spannungswandlungseinrichtung eine Mehrzahl von, vorzugsweise parallel zueinander geschalteten, H-Brückenschaltungen, insbesondere zwei oder drei H-Brückenschaltungen, umfassen, wobei die H-Brückenschaltungen jeweils vier elektronische Schalter und einen Brückenzweig, an welchem jeweils ein Elektrodenpaar des Instruments anschließbar sein kann, aufweisen können, wobei mittels des Rechteckgenerators die Schalter ansteuerbar sein können, derart, dass in den jeweiligen Brückenzweigen bzw. zwischen jeweiligen Elektroden des jeweiligen Elektrodenpaars, vorzugsweise unabhängig voneinander, die hochfrequente Rechteckspannung ausbildbar sein kann. Die hochfrequente Rechteckspannung kann an jedem der Brückenzweige abgegriffen werden. Mit einem geringen Aufwand kann somit eine, insbesondere in das Gewebe eingebrachte, abgegriffene Leistung bei einer Mehrzahl von Brückenzweigen bzw. Elektrodenpaaren gleichzeitig und unabhängig voneinander gesteuert und ggf. gemessen werden. Diese Ausführungsform kann vorteilhaft für insbesondere ein elektrochirurgisches Instrument verwendet werden, wie es in der am Prioritätstag der vorliegenden Patentanmeldung noch nicht veröffentlichten deutschen Patentanmeldung des Anmelders mit dem amtlichen Aktenzeichen 10 2022 125 714.2 beschrieben wird. Eine Anzahl der H-Brückenschaltungen kann einer Anzahl der Elektrodenpaare entsprechen, so dass jedem Elektrodenpaar genau eine H-Brückenschaltung zugeordnet sein kann. Weiter ist es denkbar, dass jede H-Brückenschaltung mittels eines eigenen Rechteckgenerators angesteuert werden kann. Eine Anzahl der Rechteckgeneratoren kann einer Anzahl der H-Brückenschaltungen entsprechen, so dass jeder H-Brückenschaltung genau ein Rechteckgenerator zugeordnet sein kann.

Vorteilhafterweise können die Schalter als Transistoren, insbesondere Bipolartransistoren oder Feldeffekttransistoren (FET), insbesondere Isolierschicht-Feldeffekttransistoren (IGFET), insbesondere Metall-Oxid-Halbleiter-Feldeffekttransistoren (MOSFET), ausgebildet sein.

Vorteilhafterweise kann der Rechteckgenerator zur Erzeugung von pulsweitenmodulierten (PWM) Signalen eingerichtet sein. Durch eine Variation eines Tastgrades und einer Frequenz der pulsweitenmodulierten Signale kann eine Frequenz der Rechteckspannung und eine in das Gewebe eingebrachte Leistung dann besonders einfach geändert werden.

Die Arbeitsspannung kann grundsätzlich an die jeweilige Hochfrequenz-Anwendung angepasst sein. Vorteilhafterweise kann die Arbeitsspannung wenigstens 100 V, bevorzugt wenigstens 200 V, besonders bevorzugt wenigstens 300 V, betragen, um die Gewebeversiegelung zu ermöglichen. Die Arbeitsspannung kann insbesondere auch 300 V betragen. Bei der Arbeitsspannung handelt es sich um eine Gleichspannung.

Vorteilhafterweise kann an der H-Brückenschaltung zusätzlich zu der an der H-Brückenschaltung anlegbaren Arbeitsspannung eine Versorgungsspannung anlegbar sein, welche einen für einen Menschen ungefährlichen Wert besitzen kann. Bei der Versorgungsspannung kann es sich um eine Gleichspannung handeln. Die Versorgungsspannung kann klein gegenüber der Arbeitsspannung sein. Beispielsweise kann die Versorgungsspannung 9 V betragen. Zur Erzeugung der Versorgungsspannung kann parallel zu der H-Brückenschaltung eine Gleichspannungsquelle, welche Bestandteil des Sekundärstromkreises sein kann, geschaltet sein. Vorzugsweise kann die Gleichspannungsquelle mittels einer Diode des Sekundärstromkreises vor Beschädigungen durch die vergleichsweise hohe Arbeitsspannung geschützt werden.

Eine Frequenz der hochfrequenten Rechteckspannung kann grundsätzlich an die jeweilige Hochfrequenz-Anwendung angepasst sein. Vorteilhafterweise kann die hochfrequente Rechteckspannung eine Frequenz von wenigstens 150 kHz, bevorzugt von wenigstens 200 kHz, besonders bevorzugt von wenigstens 300 kHz, besitzen. Eine Stimulation von Nerven eines Patienten kann dadurch ausgeschlossen werden.

Vorteilhafterweise kann der Hochfrequenzgenerator einen Wechselspannungsgenerator zur Erzeugung der Wechselspannung umfassen. Der Wechselspannungsgenerator, der Übertrager und der Gleichrichter können einen Gleichspannungsgenerator ausbilden.

In einer vorteilhaften Ausführungsform der Erfindung kann der Wechselspannungsgenerator eine in den Primärstromkreis integrierte H-Brückenschaltung umfassen, an welche eine Versorgungsspannung anlegbar sein kann, wobei die H-Brückenschaltung vier elektronische Schalter aufweisen kann, wobei der Wechselspannungsgenerator eine Ansteuereinrichtung umfassen kann, welche zur Ansteuerung der Schalter eingerichtet sein kann, derart, dass die Wechselspannung erzeugbar sein kann. In vorteilhafter Weise eröffnet diese Ausbildung des Wechselspannungsgenerators die Möglichkeit, besonders einfach ein Sicherungssystem, welches weiter unten noch beschrieben wird, bei dem Hochfrequenzgenerator vorsehen zu können. Die Schalter können als Transistoren, insbesondere Bipolartransistoren oder Feldeffekttransistoren (FET), insbesondere Isolierschicht-Feldeffekttransistoren (IGFET), insbesondere Metall-Oxid-Halbleiter-Feldeffekttransistoren (MOSFET), ausgebildet sein. Weiter kann die Versorgungsspannung, bei welcher es sich vorteilhafterweise um eine Gleichspannung handeln kann, beispielsweise 12 V bis 24 V, insbesondere 12 V, betragen. Die Versorgungsspannung kann mittels einer Spannungsquelle erzeugt werden, welche Bestandteil des Primärstromkreises sein kann. Weiter kann die Versorgungsspannung klein gegenüber der Arbeitsspannung sein. Die Ansteuereinrichtung kann eine Ansteuerlogik oder ein Treiber sein. Weiter kann die Ansteuereinrichtung eine Taktaufbereitung durchführen. Dabei kann ein Deltamodulations-Verfahren verwendet werden. Die Ansteuereinrichtung kann beispielsweise von einem Prozessor, insbesondere eines Computers, ausgebildet werden. Grundsätzlich ist es jedoch auch denkbar, eine Netzspannung an den Primärstromkreis anzulegen, wobei die Netzspannung dann optional über einen Transformator an den Primärstromkreis anlegbar sein kann.

Vorteilhafterweise kann der Hochfrequenzgenerator eine Messeinrichtung zur Messung einer in dem Sekundärstromkreis bzw. zwischen den Elektroden ausgebildeten Spannung bzw. der in dem Sekundärstromkreis bzw. zwischen den Elektroden ausgebildeten hochfrequenten Rechteckspannung und/oder eines in dem Sekundärstromkreis bzw. zwischen den Elektroden ausgebildeten Stroms umfassen, wobei dazu insbesondere die Spannung bzw. Rechteckspannung an bzw. der Strom in dem Brückenzweig erfasst werden kann. Die Messung kann direkt oder indirekt erfolgen. Beispielsweise kann als Messeinrichtung ein Zangenstrommesser vorgesehen sein. Die Messeinrichtung kann ein, insbesondere zu der Spannung oder zu dem Strom proportionales, Signal generieren.

In einer vorteilhaften Ausführungsform der Erfindung kann mittels der Messeinrichtung ein Signal ausgebbar sein, wobei der Hochfrequenzgenerator eine Verarbeitungseinrichtung umfassen kann, welche dazu eingerichtet sein kann, das Signal zu empfangen, anhand des Signals auf einen, insbesondere ordnungsgemäßen oder fehlerhaften, Betriebszustand der Spannungswandlungseinrichtung bzw. der in den Sekundärstromkreis integrierten H-Brückenschaltung zu schließen und ein den Betriebszustand beschreibendes Signal zu generieren.

Vorteilhafterweise kann dann mittels der Verarbeitungseinrichtung das den Betriebszustand beschreibende Signal ausgebbar sein, wobei der Wechselspannungsgenerator bzw. die Ansteuereinrichtung dazu eingerichtet sein kann, das den Betriebszustand beschreibende Signal zu empfangen und im Fall des ordnungsgemäßen Betriebszustands eine Erzeugung der Wechselspannung zu initiieren und/oder die Erzeugung der Wechselspannung aufrechtzuerhalten und/oder im Fall des fehlerhaften Betriebszustands die Erzeugung der Wechselspannung nicht zu initiieren und/oder die Erzeugung der Wechselspannung abzubrechen. Im Fall eines Defekts der Spannungswandlungseinrichtung bzw. der in den Sekundärstromkreis integrierten H-Brückenschaltung kann so ein Anliegen einer vergleichsweise hohen Spannung an dem Sekundärstromkreis vermieden werden. Die Messeinrichtung und die Verarbeitungseinrichtung können damit also ein Sicherungssystem des Hochfrequenzgenerators ausbilden. Eine Übertragung des mittels der Messeinrichtung ausgegebenen Signals zu der Verarbeitungseinrichtung und/oder eine Übertragung des mittels der Verarbeitungseinrichtung ausgegebenen den Betriebszustand beschreibenden Signals zu dem Wechselspannungsgenerator bzw. der Ansteuereinrichtung kann mittels eines Optokopplers erfolgen, bei welchem es sich um ein elektrisch nicht leitfähiges Kopplungsglied handelt.

In einer Ausführungsform der Erfindung kann die Verarbeitungseinrichtung ein Und-Gatter aufweisen. Dies ist insbesondere vorteilhaft, wenn die Spannungswandlungseinrichtung eine Mehrzahl von H-Brückenschaltungen umfasst, wobei dann beispielsweise an bzw. in jedem Brückenzweig die Spannung bzw. der Strom mittels der Messeinrichtung gemessen werden kann, wobei jeweilige mittels der Messeinrichtung generierte Signale an die Verarbeitungseinrichtung bzw. das Und-Gatter übermittelt werden können. Befindet sich zumindest eine H-Brückenschaltung in einem fehlerhaften Betriebszustand, beispielsweise im Fall eines Defekts, kann dies von der Verarbeitungseinrichtung bzw. dem Und-Gatter erkannt werden, wobei das von der Verarbeitungseinrichtung dann den fehlerhaften Betriebszustand beschreibende Signal den Wechselspannungsgenerator bzw. die Ansteuereinrichtung dazu veranlassen kann, die Erzeugung der Wechselspannung nicht zu initiieren bzw. die Erzeugung der Wechselspannung abzubrechen.

Das erfindungsgemäße System umfasst einen erfindungsgemäßen Hochfrequenzgenerator und ein Hochfrequenz-Gerät, insbesondere ein elektrochirurgisches Instrument zum Koagulieren und/oder Schneiden von einem biologischen Gewebe, welches an einen Sekundärstromkreis des Hochfrequenzgenerators angeschlossen ist.

Das Instrument kann zumindest ein Elektrodenpaar umfassen, welches an den Sekundärstromkreis angeschlossen sein kann, derart, dass der Sekundärstromkreis über das Gewebe schließbar sein kann.

Weiter kann das Instrument monopolar oder bipolar ausgebildet ist. Das Elektrodenpaar kann dann zumindest eine Aktivelektrode und eine Neutralelektrode oder zumindest zwei Aktivelektroden umfassen. Vorteilhafterweise ist das Instrument jedoch bipolar ausgebildet.

Bei dem erfindungsgemäßen Verfahren zur Erzeugung einer hochfrequenten Rechteckspannung mittels eines Hochfrequenzgenerators wird an einem Primärstromkreis des Hochfrequenzgenerators eine Wechselspannung angelegt, wobei ein Sekundärstromkreis des Hochfrequenzgenerators mittels eines Übertragers des Hochfrequenzgenerators von dem Primärstromkreis galvanisch getrennt an den Primärstromkreis gekoppelt ist, wobei mittels einer Spannungswandlungseinrichtung des Hochfrequenzgenerators aus der mittels des Übertragers von dem Primärstromkreis auf den Sekundärstromkreis übertragenen Wechselspannung die hochfrequente Rechteckspannung erzeugt wird.

Zu den vorteilhaften Wirkungen des erfindungsgemäßen Verfahrens wird auf die Vorteilsbeschreibung des erfindungsgemäßen Hochfrequenzgenerators verwiesen.

An dem Sekundärstromkreis kann ein Hochfrequenz-Gerät bzw. elektrochirurgisches Instrument bzw. zumindest ein Elektrodenpaar des elektrochirurgischen Instruments angeschlossen werden, wobei zwischen Elektroden des Elektrodenpaars die hochfrequente Rechteckspannung erzeugt werden kann. Die hochfrequente Rechteckspannung kann an dem Hochfrequenz-Gerät bzw. elektrochirurgischen Instrument angelegt werden.

In einer vorteilhaften Ausführungsform des Verfahrens kann mittels eines in den Sekundärstromkreis integrierten Gleichrichters der Spannungswandlungseinrichtung die mittels des Übertragers von dem Primärstromkreis auf den Sekundärstromkreis übertragene Wechselspannung in eine gleichgerichtete Arbeitsspannung umgewandelt werden, wobei die Arbeitsspannung an zumindest einer in den Sekundärstromkreis integrierten H-Brückenschaltung angelegt werden kann, wobei an einem Brückenzweig der H-Brückenschaltung das Hochfrequenz-Gerät bzw. das elektrochirurgische Instrument bzw. das Elektrodenpaar angeschlossen werden kann, wobei mittels zumindest eines Rechteckgenerators der Spannungswandlungseinrichtung vier elektronische Schalter der H-Brückenschaltung angesteuert werden können, derart, dass an dem Brückenzweig bzw. zwischen den Elektroden die hochfrequente Rechteckspannung ausgebildet werden kann.

Vorteilhafterweise kann bei einer Inbetriebnahme des Hochfrequenzgenerators bzw. Hochfrequenz-Geräts bzw. elektrochirurgischen Instruments an der in den Sekundärstromkreis integrierten H-Brückenschaltung zunächst eine Versorgungsspannung angelegt werden, welche einen für einen Menschen ungefährlichen Wert besitzen kann, wobei mittels einer Messeinrichtung des Hochfrequenzgenerators eine in dem Brückenzweig bzw. zwischen den Elektroden ausgebildete Spannung und/oder ein in dem Brückenzweig bzw. zwischen den Elektroden ausgebildeter Strom gemessen werden kann, wobei mittels der Messeinrichtung ein Signal ausgegeben werden kann, wobei mittels einer Verarbeitungseinrichtung des Hochfrequenzgenerators das Signal empfangen, anhand des Signals auf einen ordnungsgemäßen oder fehlerhaften Betriebszustand der in den Sekundärstromkreis integrierten H-Brückenschaltung geschlossen und ein den Betriebszustand beschreibendes Signal generiert werden kann, wobei mittels der Verarbeitungseinrichtung das den Betriebszustand beschreibende Signal ausgegeben werden kann, wobei mittels einer Ansteuereinrichtung eines Wechselspannungsgenerators des Hochfrequenzgenerators das den Betriebszustand beschreibende Signal empfangen werden kann und vier elektronische Schalter einer in den Primärstromkreis integrierten H-Brückenschaltung des Wechselspannungsgenerators in Abhängigkeit des den Betriebszustand beschreibenden Signals angesteuert werden können, derart, dass im Fall des ordnungsgemäßen Betriebszustands eine Erzeugung der Wechselspannung initiiert und/oder im Fall des fehlerhaften Betriebszustands die Erzeugung der Wechselspannung nicht initiiert werden kann.

Vorteilhafterweise kann nach der Inbetriebnahme bei einem Betrieb des Hochfrequenzgenerators bzw. Hochfrequenz-Geräts bzw. elektrochirurgischen Instruments an der in den Sekundärstromkreis integrierten H-Brückenschaltung zusätzlich zu der Versorgungsspannung die Arbeitsspannung angelegt werden, wobei mittels der Ansteuereinrichtung die vier elektronischen Schalter der in den Primärstromkreis integrierten H-Brückenschaltung in Abhängigkeit des den Betriebszustand beschreibenden Signals angesteuert werden können, derart, dass im Fall des ordnungsgemäßen Betriebszustands die Erzeugung der Wechselspannung aufrechterhalten und/oder im Fall des fehlerhaften Betriebszustands die Erzeugung der Wechselspannung abgebrochen werden kann, so dass nach einem Abbruch der Erzeugung der Wechselspannung an der in den Sekundärstromkreis integrierten H-Brückenschaltung nur noch die Versorgungsspannung anliegen kann. Im Fall eines Defekts kann die an dem Sekundärstromkreis anliegende Spannung also auf die für einen Menschen ungefährlichen Wert einbrechen.

Weitere vorteilhafte Ausführungsformen des Verfahrens ergeben sich aus den Merkmalsbeschreibungen der auf den Vorrichtungsanspruch 1 rückbezogenen Unteransprüche.

Erfindungsgemäß wird ein erfindungsgemäßer Hochfrequenzgenerator für ein elektrochirurgisches Instrument zum Koagulieren und/oder Schneiden von einem biologischen Gewebe verwendet.

Zu den vorteilhaften Wirkungen der erfindungsgemäßen Verwendung wird auf die Vorteilsbeschreibung des erfindungsgemäßen Hochfrequenzgenerators verwiesen.

Weitere vorteilhafte Ausführungsformen der Verwendung ergeben sich aus den Merkmalsbeschreibungen der auf den Vorrichtungsanspruch 1 rückbezogenen Unteransprüche. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Nachfolgend werden bevorzugte Ausführungsformen der Erfindung unter Bezugnahme auf die beigefügten Zeichnungen näher erläutert.

Es zeigen:
- **Fig. 1**: einen Schaltplan eines Hochfrequenzgenerators in einer Ausführungsform;
- **Fig. 2**: ein Blockschaltbild eines Hochfrequenzgenerators in einer weiteren Ausführungsform.

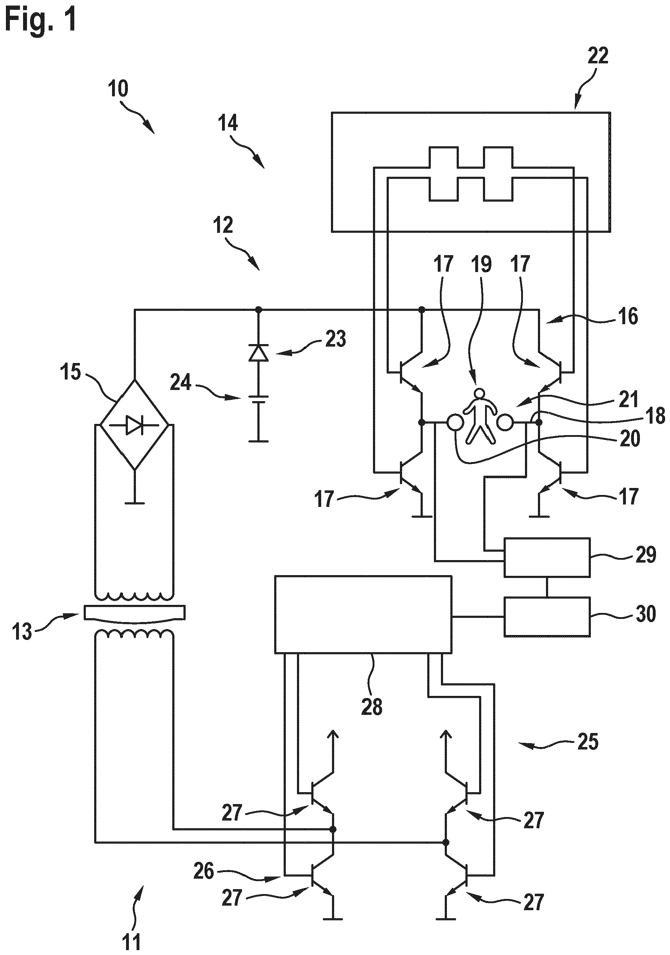

Die **Fig. 1** zeigt einen Schaltplan eines Hochfrequenzgenerators 10 für ein hier nicht näher gezeigtes elektrochirurgisches Instrument zum Koagulieren bzw. Schneiden von einem biologischen Gewebe, umfassend einen Primärstromkreis 11, an welchem eine Wechselspannung anlegbar ist, einen Sekundärstromkreis 12, einen Übertrager 13 zur von dem Sekundärstromkreis 12 galvanisch getrennten Kopplung des Primärstromkreises 11 an den Sekundärstromkreis 12 und eine Spannungswandlungseinrichtung 14, welche dazu eingerichtet ist, aus der mittels des Übertragers 13 von dem Primärstromkreis 11 auf den Sekundärstromkreis 12 übertragenen Wechselspannung eine hochfrequente Rechteckspannung, welche eine Frequenz von wenigstens 150 kHz besitzt, zu erzeugen.

Die Spannungswandlungseinrichtung 14 umfasst einen in den Sekundärstromkreis 12 integrierten Gleichrichter 15 zur Umwandlung der mittels des Übertragers 13 von dem Primärstromkreis 11 auf den Sekundärstromkreis 12 übertragenen Wechselspannung in eine gleichgerichtete Arbeitsspannung und eine in den Sekundärstromkreis 12 integrierte H-Brückenschaltung 16, an welcher die Arbeitsspannung anlegbar ist, wobei die H-Brückenschaltung 16 vier als Transistoren ausgebildete elektronische Schalter 17 und einen Brückenzweig 18, an welchem zur Behandlung eines Patienten 19 vorgesehene Elektroden 20 eines Elektrodenpaars 21 des Instruments angeschlossen sind, aufweist, wobei die Spannungswandlungseinrichtung 14 einen Rechteckgenerator 22 umfasst, welcher zur Ansteuerung der Schalter 17 eingerichtet ist, derart, dass zwischen den Elektroden 20 die hochfrequente Rechteckspannung ausbildbar ist. Dabei ist der Rechteckgenerator 22 zur Erzeugung von pulsweitenmodulierten (PWM) Signalen eingerichtet, wodurch die Ansteuerung der Schalter 17 erfolgt.

Im vorliegenden Beispiel beträgt die Arbeitsspannung 300 V. Zusätzlich zu der an der H-Brückenschaltung 16 anlegbaren Arbeitsspannung ist an der H-Brückenschaltung 16 über eine mittels einer Diode 23 vor der Arbeitsspannung geschützten Spannungsquelle 24 eine Versorgungsspannung anlegbar, welche einen für einen Menschen ungefährlichen Wert besitzt, welcher im vorliegenden Beispiel 9 V beträgt.

Weiter umfasst der Hochfrequenzgenerator 10 einen Wechselspannungsgenerator 25 zur Erzeugung der Wechselspannung, welcher eine in den Primärstromkreis 11 integrierte H-Brückenschaltung 26 umfasst, an welche eine Versorgungsspannung anlegbar ist, welche im vorliegenden Beispiel 12 V beträgt, wobei die H-Brückenschaltung 26 vier als Transistoren ausgebildete elektronische Schalter 27 aufweist, wobei der Wechselspannungsgenerator 25 eine Ansteuereinrichtung 28 umfasst, welche zur Ansteuerung der Schalter 27 eingerichtet ist, derart, dass die Wechselspannung erzeugbar ist.

Ferner umfasst der Hochfrequenzgenerator 10 eine Messeinrichtung 29 zur Messung einer zwischen den Elektroden 20 ausgebildeten Spannung bzw. eines zwischen den Elektroden 20 ausgebildeten Stroms. Mittels der Messeinrichtung 29 ist ein Signal ausgebbar, wobei der Hochfrequenzgenerator 10 eine Verarbeitungseinrichtung 30 umfasst, welche dazu eingerichtet ist, das Signal zu empfangen, anhand des Signals auf einen, insbesondere ordnungsgemäßen oder fehlerhaften, Betriebszustand der Spannungswandlungseinrichtung 14, insbesondere auf einen Defekt der H-Brückenschaltung 16, zu schließen und ein den Betriebszustand beschreibendes Signal zu generieren. Mittels der Verarbeitungseinrichtung 30 ist das den Betriebszustand beschreibende Signal ausgebbar, wobei der Wechselspannungsgenerator 25 bzw. die Ansteuereinrichtung 28 dazu eingerichtet ist, das den Betriebszustand beschreibende Signal zu empfangen und im Fall des ordnungsgemäßen Betriebszustands eine Erzeugung der Wechselspannung zu initiieren bzw. die Erzeugung der Wechselspannung aufrechtzuerhalten bzw. im Fall des fehlerhaften Betriebszustands die Erzeugung der Wechselspannung nicht zu initiieren bzw. die Erzeugung der Wechselspannung abzubrechen. Insbesondere die Anbindung der Messeinrichtung 29 an die Verarbeitungseinrichtung 30 und die Anbindung der Verarbeitungseinrichtung 30 an die Ansteuereinrichtung 28 ist hier nur schematisch dargestellt.

Der Hochfrequenzgenerator 10 wurde vorstehend im Zusammenhang mit einem elektrochirurgischen Instrument beschrieben. Jedoch kann der Hochfrequenzgenerator 10 grundsätzlich auch im Zusammenhang mit anderen Hochfrequenz-Anwendungen eingesetzt werden. Insbesondere kann an den Brückenzweig 18 ein sonstiges Hochfrequenz-Gerät angeschlossen werden.

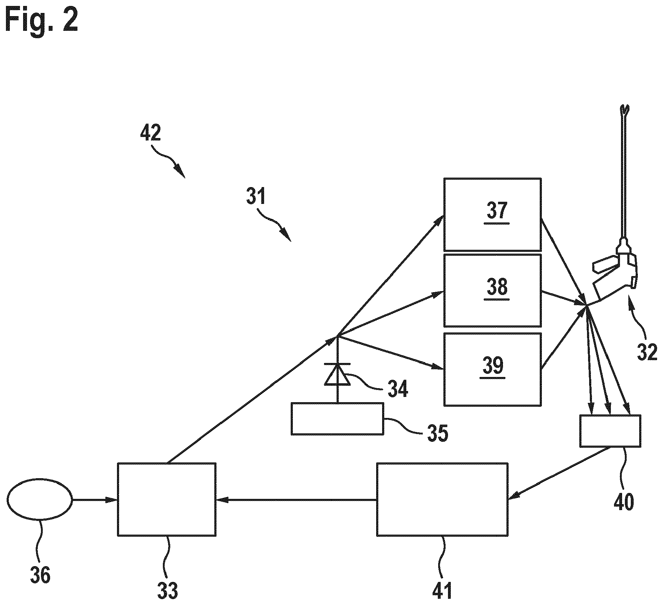

Die Fig. 2 zeigt ein Blockschaltbild eines Hochfrequenzgenerators 31 für ein elektrochirurgisches Instrument 32 zum Koagulieren bzw. Schneiden von einem biologischen Gewebe. Eine von einem Gleichspannungsgenerator 33 des Hochfrequenzgenerators 31, wobei der Gleichspannungsgenerator 33 insbesondere von einem Wechselspannungsgenerator, einem Übertrager und einem Gleichrichter ausgebildet sein kann, erzeugte Arbeitsspannung, welche im vorliegenden Beispiel 400 V beträgt, ist zusammen mit einer von einer mit einer Diode 34 vor der Arbeitsspannung geschützten Spannungsquelle 35 gelieferten Versorgungsspannung, welche im vorliegenden Beispiel 9 V beträgt, an einer hier nicht näher gezeigten Spannungswandlungseinrichtung des Hochfrequenzgenerators 31 anlegbar. Der Gleichspannungsgenerator 33 wird von einer Spannungsquelle 36, welche im vorliegenden Beispiel eine Versorgungsspannung von 12 V bis 24 V liefert, gespeist. Die Spannungswandlungseinrichtung umfasst drei hier nicht gezeigte H-Brückenschaltungen, wobei jede H-Brückenschaltung mittels eines eigenen Rechteckgenerators 37, 38, 39 der Spannungswandlungseinrichtung, welche jeweils zur Erzeugung von pulsweitenmodulierten (PWM) Signalen mit einer Frequenz von 300 kHz eingerichtet sind, jeweils zur Erzeugung einer hochfrequenten Rechteckspannung aus einer an der jeweiligen H-Brückenschaltung anlegbaren Spannung unabhängig von den jeweils beiden anderen H-Brückenschaltungen ansteuerbar ist. Jede der drei unabhängig voneinander regelbaren hochfrequenten Rechteckspannungen ist an einem jeweiligen Elektrodenpaar von drei hier nicht gezeigten Elektrodenpaaren des Instruments 32 anlegbar. Insbesondere mittels eines Und-Gatters 40 einer hier nicht näher gezeigten Verarbeitungseinrichtung des Hochfrequenzgenerators 31 wird anhand von drei mittels einer hier nicht gezeigten Messeinrichtung des Hochfrequenzgerators 31 generierten Signalen, welche aus einer Messung einer Spannung bzw. eines Stroms an bzw. in jeweiligen Brückenzweigen der H-Brückenschaltungen resultieren, auf einen ordnungsgemäßen oder fehlerhaften Betriebszustand der Spannungswandlungseinrichtung bzw. der H-Brückenschaltungen geschlossen, wobei die Verarbeitungseinrichtung ein den Betriebszustand beschreibendes Signal generiert und dieses an eine Ansteuereinrichtung 41 des Gleichspannungsgenerators 33, welche in der **Fig. 2** durch einen von einem den Gleichspannungsgenerator 33 verdeutlichenden Block getrennten, eigenen Block verdeutlicht ist, übermittelt, welche entsprechend dem den Betriebszustand beschreibenden Signal eine Erzeugung der Arbeitsspannung initiiert, aufrechterhält oder abbricht.

Der Hochfrequenzgenerator 31 und das Instrument 32 sind Bestandteil eines Systems 42.

Der Hochfrequenzgenerator 31 wurde vorstehend im Zusammenhang mit einem elektrochirurgischen Instrument bzw. dem elektrochirurgischen Instrument 32 beschrieben. Jedoch kann der Hochfrequenzgenerator 31 grundsätzlich auch im Zusammenhang mit anderen Hochfrequenz-Anwendungen bzw. sonstigen Hochfrequenz-Geräten eingesetzt werden.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die Erfindung betrifft einen Hochfrequenzgenerator 10), umfassend einen Primärstromkreis 11), an welchem eine Wechselspannung anlegbar ist, einen Sekundärstromkreis 12), einen Übertrager 13) zur von dem Sekundärstromkreis galvanisch getrennten Kopplung des Primärstromkreises an den Sekundärstromkreis und eine Spannungswandlungseinrichtung 14), welche dazu eingerichtet ist, aus der mittels des Übertragers von dem Primärstromkreis auf den Sekundärstromkreis übertragenen Wechselspannung eine hochfrequente Rechteckspannung zu erzeugen.

### Bezugszeichenliste

- 10: Hochfrequenzgenerator
- 11: Primärstromkreis
- 12: Sekundärstromkreis
- 13: Übertrager
- 14: Spannungswandlungseinrichtung
- 15: Gleichrichter
- 16: H-Brückenschaltung
- 17: elektronische Schalter
- 18: Brückenzweig
- 19: Patient
- 20: Elektrode
- 21: Elektrodenpaar
- 22: Rechteckgenerator
- 23: Diode
- 24: Spannungsquelle
- 25: Wechselspannungsgenerator
- 26: H-Brückenschaltung
- 27: elektronischer Schalter
- 28: Ansteuereinrichtung
- 29: Messeinrichtung
- 30: Verarbeitungseinrichtung
- 31: Hochfrequenzgenerator
- 32: elektrochirurgisches Instrument
- 33: Gleichspannungsgenerator
- 34: Diode
- 35: Spannungsquelle
- 36: Spannungsquelle
- 37: Rechteckgenerator
- 38: Rechteckgenerator
- 39: Rechtgenerator
- 40: Und-Gatter
- 41: Ansteuereinrichtung
- 42: System

## Patentansprüche

1. Hochfrequenzgenerator (10, 31), umfassend einen Primärstromkreis (11), an welchem eine Wechselspannung anlegbar ist, einen Sekundärstromkreis (12), einen Übertrager (13) zur von dem Sekundärstromkreis galvanisch getrennten Kopplung des Primärstromkreises an den Sekundärstromkreis und eine Spannungswandlungseinrichtung (14), welche dazu eingerichtet ist, aus der mittels des Übertragers von dem Primärstromkreis auf den Sekundärstromkreis übertragenen Wechselspannung eine hochfrequente Rechteckspannung zu erzeugen.

2. Hochfrequenzgenerator nach Anspruch 1,
**dadurch gekennzeichnet,**
**dass** an dem Sekundärstromkreis (12) ein Hochfrequenz-Gerät, insbesondere ein elektrochirurgisches Instrument (32) zum Koagulieren und/oder Schneiden von einem biologischen Gewebe, anschließbar ist, so dass die hochfrequente Rechteckspannung an dem Hochfrequenz-Gerät anlegbar ist.

3. Hochfrequenzgenerator nach Anspruch 1 oder 2,
**dadurch gekennzeichnet,**
**dass** die Spannungswandlungseinrichtung (14) einen in den Sekundärstromkreis (12) integrierten Gleichrichter (15) zur Umwandlung der mittels des Übertragers (13) von dem Primärstromkreis (11) auf den Sekundärstromkreis übertragenen Wechselspannung in eine gleichgerichtete Arbeitsspannung und zumindest eine in den Sekundärstromkreis integrierte H-Brückenschaltung (16), an welcher die Arbeitsspannung anlegbar ist, umfasst, wobei die H-Brückenschaltung vier elektronische Schalter (17) und einen Brückenzweig (18) aufweist, wobei die Spannungswandlungseinrichtung zumindest einen Rechteckgenerator (22, 37, 38, 39) umfasst, welcher zur Ansteuerung der Schalter eingerichtet ist, derart, dass in dem Brückenzweig die hochfrequente Rechteckspannung ausbildbar ist.

4. Hochfrequenzgenerator nach Anspruch 3,
**dadurch gekennzeichnet,**
**dass** die Spannungswandlungseinrichtung (14) eine Mehrzahl von, vorzugsweise parallel zueinander geschalteten, H-Brückenschaltungen (16), insbesondere zwei oder drei H-Brückenschaltungen, umfasst, wobei die H-Brückenschaltungen jeweils vier elektronische Schalter (17) und einen Brückenzweig (18) aufweisen, wobei mittels des Rechteckgenerators (22, 37, 38, 39) die Schalter ansteuerbar sind, derart, dass in den jeweiligen Brückenzweigen, vorzugsweise unabhängig voneinander, die hochfrequente Rechteckspannung ausbildbar ist.

5. Hochfrequenzgenerator nach einem der Ansprüche 3 oder 4,
**dadurch gekennzeichnet,**
**dass** der Rechteckgenerator (22, 37, 38, 39) zur Erzeugung von pulsweitenmodulierten (PWM) Signalen eingerichtet ist.

6. Hochfrequenzgenerator nach einem der Ansprüche 3 bis 5,
**dadurch gekennzeichnet,**
**dass** die Arbeitsspannung wenigstens 100 V, bevorzugt wenigstens 200 V, besonders bevorzugt wenigstens 300 V, beträgt.

7. Hochfrequenzgenerator nach einem der Ansprüche 3 bis 6,
**dadurch gekennzeichnet,**
**dass** an der H-Brückenschaltung (16) zusätzlich zu der an der H-Brückenschaltung anlegbaren Arbeitsspannung eine Versorgungsspannung anlegbar ist.

8. Hochfrequenzgenerator nach einem der vorangehenden Ansprüche,
**dadurch gekennzeichnet,**
**dass** die hochfrequente Rechteckspannung eine Frequenz von wenigstens 150 kHz, bevorzugt von wenigstens 200 kHz, besonders bevorzugt von wenigstens 300 kHz, besitzt.

9. Hochfrequenzgenerator nach einem der vorangehenden Ansprüche,
**dadurch gekennzeichnet,**
**dass** der Hochfrequenzgenerator (10, 31) einen Wechselspannungsgenerator (25) zur Erzeugung der Wechselspannung umfasst.

10. Hochfrequenzgenerator nach Anspruch 9,
**dadurch gekennzeichnet,**
**dass** der Wechselspannungsgenerator (25) eine in den Primärstromkreis (11) integrierte H-Brückenschaltung (26) umfasst, an welcher eine Versorgungsspannung anlegbar ist, wobei die H-Brückenschaltung vier elektronische Schalter (27) aufweist, wobei der Wechselspannungsgenerator eine Ansteuereinrichtung (28, 41) umfasst, welche zur Ansteuerung der Schalter eingerichtet ist, derart, dass die Wechselspannung erzeugbar ist.

11. Hochfrequenzgenerator nach einem der vorangehenden Ansprüche,
**dadurch gekennzeichnet,**
**dass** der Hochfrequenzgenerator (10, 31) eine Messeinrichtung (29) zur Messung einer in dem Sekundärstromkreis (12) ausgebildeten Spannung und/oder eines in dem Sekundärstromkreis ausgebildeten Stroms umfasst.

12. Hochfrequenzgenerator zumindest nach Anspruch 9 und 11,
**dadurch gekennzeichnet,**
**dass** mittels der Messeinrichtung (29) ein Signal ausgebbar ist, wobei der Hochfrequenzgenerator (10, 31) eine Verarbeitungseinrichtung (30) umfasst, welche dazu eingerichtet ist, das Signal zu empfangen, anhand des Signals auf einen, insbesondere ordnungsgemäßen oder fehlerhaften, Betriebszustand der Spannungswandlungseinrichtung (14) zu schließen und ein den Betriebszustand beschreibendes Signal zu generieren.

13. Hochfrequenzgenerator nach Anspruch 12,
**dadurch gekennzeichnet,**
**dass** mittels der Verarbeitungseinrichtung (30) das den Betriebszustand beschreibende Signal ausgebbar ist, wobei der Wechselspannungsgenerator (25) dazu eingerichtet ist, das den Betriebszustand beschreibende Signal zu empfangen und im Fall des ordnungsgemäßen Betriebszustands eine Erzeugung der Wechselspannung zu initiieren und/oder die Erzeugung der Wechselspannung aufrechtzuerhalten und/oder im Fall des fehlerhaften Betriebszustands die Erzeugung der Wechselspannung nicht zu initiieren und/oder die Erzeugung der Wechselspannung abzubrechen.

14. System (42), umfassend einen Hochfrequenzgenerator (10, 31) nach einem der vorangehenden Ansprüche und ein Hochfrequenz-Gerät, insbesondere ein elektrochirurgisches Instrument (32) zum Koagulieren und/oder Schneiden von einem biologischen Gewebe, welches an einen Sekundärstromkreis (12) des Hochfrequenzgenerators angeschlossen ist.

15. Verfahren zur Erzeugung einer hochfrequenten Rechteckspannung mittels eines Hochfrequenzgenerators (10, 31), wobei an einem Primärstromkreis (11) des Hochfrequenzgenerators eine Wechselspannung angelegt wird, wobei ein Sekundärstromkreis (12) des Hochfrequenzgenerators mittels eines Übertragers (13) des Hochfrequenzgenerators von dem Primärstromkreis galvanisch getrennt an den Primärstromkreis gekoppelt ist, wobei mittels einer Spannungswandlungseinrichtung (14) des Hochfrequenzgenerators aus der mittels des Übertragers von dem Primärstromkreis auf den Sekundärstromkreis übertragenen Wechselspannung die hochfrequente Rechteckspannung erzeugt wird.
